# EUROPEAN PATENT SPECIFICATION

(11) **EP 0 642 574 B1**
(45) Date of publication and mention of the grant of the patent: **09.12.1998**
(21) Application number: 92918801.9
(22) Date of filing: 26.08.1992
(51) Int. Cl.: C11D 3/386

(54) **DETERGENT COMPOSITIONS**
WASCHMITTELZUSAMMENSETZUNGEN
COMPOSITIONS POUR DETERGENTS

(30) Priority: 27.08.1991 WO PCT/DK91/00249
(43) Date of publication of application: 15.03.1995
(73) Proprietor: NOVO NORDISK A/S, 2880 Bagsvaerd (DK)
(72) Inventor: OUTTRUP, Helle, DK-2750 Ballerup (DK); AASLYNG, Dorrit, DK-4000 Roskilde (DK); DAMBMANN, Claus, DK-2860 Soeborg (DK); PATKAR, Shamkant, Anant, DK-2800 Lyngby (DK)
(74) Representative: Knudsen, Sten Lottrup
(86) International application number: DK9200251
(87) International publication number: WO9304157

(56) References cited:
- EP-A- 0 245 560
- GB-A- 1 029 978
- Biochim.Biophys.Acta, Vol.110, p.585-598 & p.599-607

## Description

### TECHNICAL FIELD

This invention relates to the use of proteases for detergent purposes. More specifically, the invention relates to the use of proteases derived from members of the genus Arthrobacter for detergent purposes.

### BACKGROUND ART

Microorganisms belonging to the genus Arthrobacter are well known in the literature and they are known to produce various enzymes, e.g. endodextranases, aminopeptidases, glucoseisomerases, cephalosporin C amidase, and adenylcobamide coenzyme, as well as other compounds, e.g. polysaccharides. Various applications for these and other compounds have been described.

Proteolytic enzymes from strains of Arthrobacter are also well known and have been thoroughly studied, vide e.g. Hofsten et al. [Biochim. Biophys. Acta 110 (1965); 576-584; 585-598; and 599-607].

In GB Patent Specification No. 1,029,978 it is proposed to use a proteolytic enzyme from Arthrobacter in the foodstuff industry, in leather and textile processing, and in basic scientific research.

In EP Patent Application No. 245,560 it is proposed to use viable microorganisms, e.g. bacterial cells from strains of Arthrobacter in abrasive surface cleaners, in order to improve waste degradation and facilitate sewage treatment, due to the microbial growth.

Surprisingly, it has now been found that excellent washing properties are conferred to detergent compositions when proteolytic enzymes obtainable from strains of the genus Arthrobacter are incorporated into these compositions.

### SUMMARY OF THE INVENTION

The present invention relates to the use of proteolytic enzymes obtainable from strains of the genus Arthrobacter for detergent purposes.

Accordingly, the invention provides detergent compositions comprising one or more proteases obtainable from, or one or more proteases having immunochemical properties identical or partially identical to those of a protease derived from any of the strains NCIMB No. 40294; NCIMB No. 40295; NCIMB No. 40296; NCIMB No. 40297; NCIMB No. 40298; NCIMB No. 40299; NCIMB 40300; and ATCC No. 7562.

In another aspect, the invention provides detergent compositions comprising one or more proteases obtainable from a member of the genus Arthrobacter, or a mutant or a variant thereof.

In yet another aspect, the invention provides detergent additives comprising a protease obtainable from, or a protease having immunochemical properties identical or partially identical to those of a protease derived from any of the strains NCIMB No. 40294; NCIMB No. 40295; NCIMB No. 40296; NCIMB No. 40297; NCIMB No. 40298; NCIMB No. 40299; NCIMB 40300; and ATCC No. 7562.

In a further aspect, the invention provides detergent additives comprising a protease obtainable from a member of the genus Arthrobacter, or a mutant or a variant thereof.

### BRIEF DESCRIPTION OF DRAWINGS

The present invention is further illustrated by reference to the accompanying drawings, in which:
Fig. 1 shows the relation between temperature (°C) and proteolytic activity (% relative) of an enzyme of the invention (■ at pH 9.5; □ at pH 9.5 with 0.1% STPP added); and
Fig. 2 shows the relation between pH and proteolytic activity (% relative) of an enzyme of the invention.

### DETAILED DISCLOSURE OF THE INVENTION

The present invention provides detergent compositions comprising one or more protease(s) obtainable from a strain of the genus Arthrobacter. Microorganisms belonging to the genus Arthrobacter are well known and described in the literature.

Strains of Arthrobacter have been deposited and made available from various international depositary institutes, e.g. NCIMB, No. 40294; NCIMB, No. 40295; NCIMB, No. 40296; NCIMB, No. 40297; NCIMB, No. 40298; NCIMB, No. 40299; NCIMB, No. 40300; and ATCC No. 7562.

The proteases are obtainable by methods known and described in the literature, e.g. by cultivation of a protease producing strain of the genus Arthrobacter in a suitable nutrient medium, containing carbon and nitrogen sources and inorganic salts, followed by recovery of the desired enzyme, or may e.g. be produced by employing recombinant DNA technology.

### The Proteases

In the context of this invention, suitable proteases are the proteases obtainable from strains of Arthrobacter, or mutants or variants thereof, or proteases having immunochemical properties identical or partially identical to a protease obtainable from a strain of Arthrobacter.

By an enzyme variant or mutated enzyme is meant an enzyme obtainable by alteration of the DNA nucleotide sequence of the parent gene or its derivatives. The enzyme variant or mutated enzyme may be expressed and produced when the DNA nucleotide sequence encoding the enzyme is inserted into a suitable vector in a suitable host organism. The host organism is not necessarily identical to the organism from which the parent gene originated.

The immunochemical properties can be determined immunologically by cross-reaction identity tests. The identity tests can be performed by the well-known Ouchterlony double immunodiffusion procedure or by tandem crossed immunoelectrophoresis according to N. H. Axelsen; Handbook of Immuno-precipitation-in-Gel Techniques; Blackwell Scientific Publications (1983), chapters 5 and 14. The terms "antigenic identity" and "partial antigenic identity" are described in the same book, chapters 5, 19 and 20.

### Detergent Compositions

The detergent composition of the invention may comprise one or more surfactants, which may be of an anionic, non-ionic, cat-ionic, amphoteric or zwitterionic type, or a mixture of these. Typical examples of anionic surfactants are linear alkyl benzene sulfonates (LAS); alkyl sulfates (AS); alpha olefin sulfonates (AOS); alcohol ethoxy sulfates (AES) and alkali metal salts of natural fatty acids. Examples of non-ionic surfactants are alkyl polyethylene glycol ethers; nonylphenol polyethylene glycol ethers; fatty acids esters of sucrose and glucose; and esters of polyethoxylated alkyl glucoside.

The detergent composition of the invention may also contain other detergent ingredients known in the art such as builders, bleaching agents, bleach activators, anti-corrosion agents, sequestering agents, anti soil-redeposition agents, perfumes, stabilizers for the enzymes and bleaching agents, formulations aids, optical brighteners, foam boosters, chelating agents, fillers, fabric softeners, etc. The detergent composition of the invention may be formulated substantially as described in J. Falbe [Falbe. J.; Surfactants in Consumer Products. Theory, Technology and Application; Springer Verlag 1987, vide in particular the section entitled "Frame formulations for liquid/powder heavy-duty detergents"].

It is at present contemplated that the detergent composition of the invention may contain the enzyme preparation in an amount corresponding to 0.0005-0.5 Anson Units (AU) of the proteolytic enzyme per litre of washing liquor.

One Anson Unit is the amount of enzyme which, under standard conditions (i.e. at 25.0°C; pH 7.50; and a reaction time of 10 minutes) digests haemoglobin at an initial rate so that there is liberated per minute an amount of trichloroacetic acid soluble product which gives the same colour with phenol reagent as one milliequivalent of tyrosine. A folder AF 4/5 describing this analytical method is available upon request to Novo Nordisk A/S, Denmark.

The detergent compositions of the invention can be formulated in any convenient form, such as powders, liquids, etc.

The detergent composition of the invention may advantageously include one or more other enzymes, e.g. lipases, amylases, cellulases, oxidases, and/or peroxidases, conventionally included in detergent compositions, as well as proteases of other origin.

The protease of the invention may be included in a detergent composition by adding separate additives containing the detergent protease, or by adding a combined additive comprising different detergent enzymes.

The additive of the invention, i.e. a separated additive or a combined additive, can be formulated e.g. as granulates, liquids, slurries, etc. Preferred detergent additive formulations are non-dusting granulates, liquids, in particular stabilized liquids, slurries, or protected enzymes. Dust free granulates may be produced according to e.g. GB Patent No. 1,362,365 or US Patent No. 4,106,991, and may optionally be coated by methods known in the art. The detergent enzymes may be mixed before or after granulation. Liquid enzyme preparations may, for instance, be stabilized by adding a polyol such as e.g. propylene glycol; a sugar or sugar alcohol; lactic acid or boric acid, according to established methods. Other enzyme stabilizers are well known in the art. Protected enzymes may be prepared according to the method disclosed in EP Patent Application No. 238,216.

The invention is further illustrated in the following examples which are not intended to be in any way limiting to the scope of the invention as claimed.

### EXAMPLE 1

### Preparation Example

Arthrobacter sp. PD61 (NCIMB 40299) was cultivated at 25°C on a rotary shaking table (300 r.p.m.) in 500 ml baffled Erlenmeyer flasks containing 100 ml of medium of the following composition (per litre):

| | |
|---|---|
| Potato starch | 100 g |
| Ground barley | 50 g |
| Soybean flour | 20 g |
| Na₂HPO₄ x 12 H₂O | 9 g |
| Pluronic® | 0.1 g |
| Sodium caseinate | 10 g |

The starch in the medium is liquified with α-amylase, and the medium is sterilized by heating at 120°C for 45 minutes.

After 5 days of incubation a proteolytic activity of the culture of 4 CPU/I was determined using the method described below.

After separation of the solid material the protease was purified by a conventional chromatographic method.

Yield from 1 l of culture broth was 10 ml with 57 CPU/I. Purity was more than 90% as judged by SDS-PAGE.

Preparations from the strains Arthrobacter sp. ME170 (NCIMB 40294); Arthrobacter sp. SJ76 (NCIMB 40300); and Arthrobacter sp. A87 (ATCC 7562), were obtained in similar ways.

### EXAMPLE 2

### Characterization of the Enzyme

The preparation prepared in accordance with Example 1 was subjected to the following characterization.

A molecular weight of 25 kD was determined by SDS-PAGE. A pl of 9.2 was determined by isoelectric focusing on LKB Ampholine® PAG plates. The protease activity is inhibited by PMSF, α-1-antitrypsin, and soybean proteinase inhibitor. EDTA and Turkey-egg-white proteinase inhibitor do not influence the protease activity.

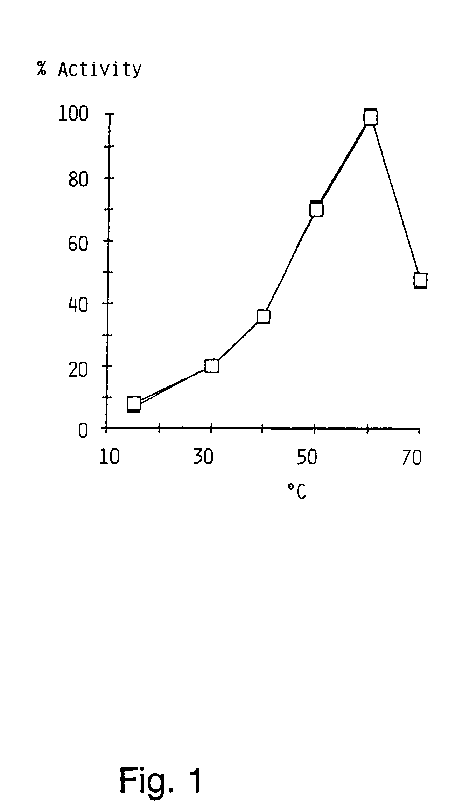

The temperature activity relationship was determined with casein as substrate. The assay for proteolytic activity described below was used with the modification that the incubation temperature was varied in the interval of from 15 to 70°C. The result is shown in Fig. 1. The enzyme possesses proteolytic activity from temperatures below 15°C to above 70°C, and a temperature optimum within the range of from 50 to 65°C; around 60°C.

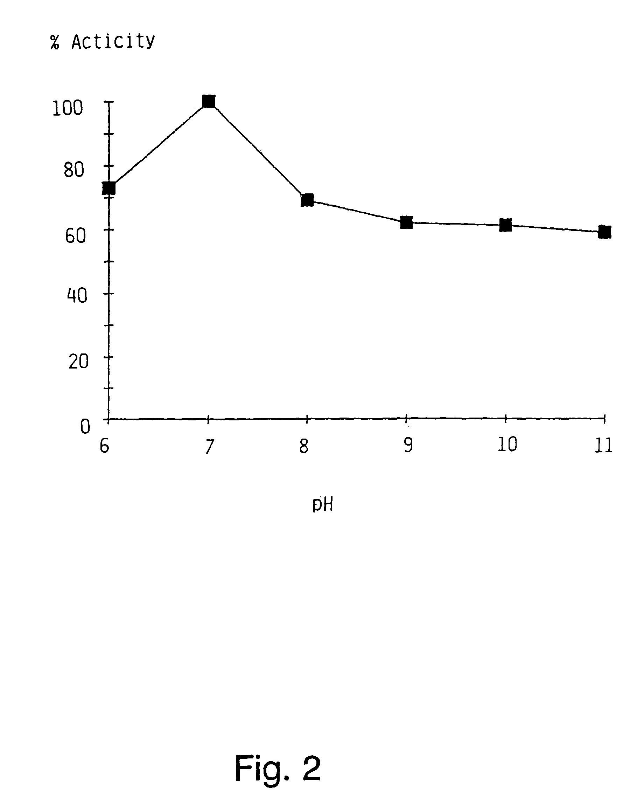

The dependence of activity on pH was determined by the same procedure, using buffers adjusted to predetermined pH values in the pH range of from 6 to 11. The result is shown in Fig. 2. The enzyme possesses proteolytic activity at pH values below 6 to above 11, with a pH optimum in the range of from pH 6 to pH 8; around pH 7.

### Assay for Proteolytic Activity

The proteolytic activity is determined with casein as substrate. One Casein Protease Unit (CPU) is defined as the amount of enzyme liberating 1 mM of primary amino groups (determined by comparison with a serine standard) per minute under standard conditions, i.e. incubation for 30 minutes at 25°C and pH 9.5. A folder AF 228, describing the analytical method, is available upon request to Novo Nordisk A/S, Denmark, which folder is hereby included by reference.

### EXAMPLE 3

### Wash Performance

Two sets of wash performance tests were accomplished on grass juice soiled cotton at 20°C, isothermally for 10 minutes.

In the first set 2.0 g/l of a commercial American type powder detergent were used. The detergent was dissolved in approx. 6° dH (German Hardness) water. The pH was 9.5. The result of these tests is shown in Table 1 below.

In the second set 2.0 g/l of a commercial American type liquid deters gent were used. The detergent was dissolved in approx. 6° dH water. The pH was 7.8. The result of these tests is shown in Tables 2 and 3 below.

In both sets the textile/wash liquor ratio was 6 g of textile per litre of wash liquor.

Subsequent to washing the cloths were rinsed in running tap-water and air-dried. The remission (%R) was determined at 460 nm.

As a measure of the wash performance differential remission, AR, was used being equal to the remission after wash with enzyme added, minus the 5 remission after wash with no enzyme added.

**Table 1**

| | | | | | | | |
|---|---|---|---|---|---|---|---|
| The differential remission, Δ R, measured after wash in a commercial American type powder detergent. | | | | | | | |

| | Enzyme dosage (AU/I) | | | | | | |
|---|---|---|---|---|---|---|---|
| | 0.004 | 0.008 | 0.02 | 0.04 | 0.08 | 0.4 | 8.0 |
| ME170 | 1.5 | 3.5 | 6.8 | 9.9 | 11.2 | 16.8 | - |
| SJ76 | 3.3 | 4.2 | 5.8 | 8.3 | 10.8 | 16.0 | 17.0 |
| PD61 | 2.3 | 3.7 | 6.0 | 8.6 | 10.7 | 16.3 | 17.6 |

**Table 2**

| | | | | | | | |
|---|---|---|---|---|---|---|---|
| The differential remission, Δ R, measured after wash in a commercial American type liquid detergent. | | | | | | | |

| | Enzyme dosage (AU/I) | | | | | | |
|---|---|---|---|---|---|---|---|
| | 0.0025 | 0.005 | 0.01 | 0.05 | 0.1 | 0.2 | 0.5 |
| ME170 | 1.8 | 4.3 | 7.3 | 18.1 | 23.0 | 24.0 | - |
| SJ76 | 2.3 | 5.2 | 9.0 | 21.0 | 23.8 | 24.1 | 24.6 |
| PD61 | 3.5 | 5.6 | 10.2 | 21.9 | 24.0 | 25.2 | 25.6 |

**Table 3**

| | | | | |
|---|---|---|---|---|
| The differential remission, Δ R, measured after wash in a commercial American type liquid detergent. | | | | |

| | Enzyme dosage (AU/I) | | | |
|---|---|---|---|---|
| | 0.019 | 0.0375 | 0.075 | 0.75 |
| A87 | 10.8 | 15.7 | 20.5 | 24.3 |

As indicated by the differential remission values the proteases of the invention are well suited for use as detergent enzymes.

## Claims

1. Use of a protease obtainable from a member of the genus Arthrobacter for detergent purpose in a washing process.

2. The use according to claim 1, wherein the protease is obtainable from the strain NCIMB No. 40294; the strain NCIMB No. 40295; the strain NCIMB No. 40296; the strain NCIMB No. 40297; the strain NCIMB No. 40298; the strain NCIMB No. 40299; the strain NCIMB No. 40300; or the strain ATCC No. 7562, or mutants of these strains.

3. The use according to claim 2, wherein the protease is obtainable from the strain NCIMB No. 40294; the strain NCIMB No. 40299; the strain NCIMB No. 40300; or the strain ATCC No. 7562, or mutants of these strains.

4. The use according to any of claims 1-3, wherein the protease is used simultaneously with one or more other enzymes from the group of amylases, lipases, cellulases, oxidases, and peroxidases.

5. The use according to any of claims 1-4, wherein the protease is provided in the form of a non-dusting granulate, a liquid, in particular a stabilized liquid, a slurry, or a protected enzyme.

## Patentansprüche

1. Verwendung einer Protease, die aus einem Mitglied der Gattung *Arthrobacter* erhältlich ist, für Waschmittelzwecke in einem Waschverfahren.

2. Verwendung nach Anspruch 1, wobei die Protease aus dem Stamm NCIMB Nr. 40294, aus dem Stamm NCIMB Nr. 40295, aus dem Stamm NCIMB Nr. 40296, aus dem Stamm NCIMB Nr. 40297, aus dem Stamm NCIMB Nr. 40298, aus dem Stamm NCIMB Nr. 40299, aus dem Stamm NCIMB Nr. 40300, aus dem Stamm ATCC Nr. 7562 oder aus Mutanten dieser Stämme erhältlich ist.

3. Verwendung nach Anspruch 2, wobei die Protease aus dem Stamm NCIMB Nr. 40294; aus dem Stamm NCIMB Nr. 40299; aus dem Stamm NCIMB Nr. 40300; aus dem Stamm ATCC Nr. 7562 oder aus Mutanten dieser Stämme erhältlich ist.

4. Verwendung nach einem der Ansprüche 1 bis 3, wobei die Protease gleichzeitig mit einem oder mehreren anderen Enzymen aus der Gruppe Amylasen, Lipasen, Cellulasen, Oxidasen und Peroxidasen verwendet wird.

5. Verwendung nach einem der Ansprüche 1 bis 4, wobei die Protease in Form eines staubfreien Granulats, einer Flüssigkeit, insbesondere einer stabilisierten Flüssigkeit, einer Aufschlämmung oder eines geschützten Enzyms bereitgestellt wird.

## Revendications

1. Utilisation d'une protéase pouvant être obtenue à partir d'un élément du genre Arthrobacter destinées à des détergents dans un processus de lavage.

2. Utilisation selon la revendication 1, dans laquelle la protéase peut être obtenue à partir de la souche NCIMB n° 40294 ; la souche NCIMB n° 40295 ; la souche NCIMB n° 40296 ; la souche NCIMB n° 40297 ; la souche NCIMB n° 40298 ; la souche NCIMB n° 40299 ; la souche NCIMB n° 40300 ; ou la souche ATCC n° 7562, ou des mutants de ces souches.

3. Utilisation selon la revendication 2, dans laquelle la protéase peut être obtenue à partir de la souche NCIMB n° 40294 ; la souche NCIMB n° 40299 ; la souche NCIMB n° 40300 ; ou la souche ATCC n° 7562, ou des mutants de ces souches.

4. Utilisation selon l'une quelconque des revendications 1 à 3, dans laquelle la protéase est utilisée simultanément avec une ou plusieurs autres enzymes provenant du groupe constitué des amylases, des lipases, des cellulases, des oxydases et des peroxydases.

5. Utilisation selon l'une quelconque des revendications 1 à 4, dans laquelle la protéase est fournie sous la forme d'un granulé non-poussiéreux, d'un liquide, en particulier un liquide stabilisé, d'une pâte, ou d'une enzyme protégée.
